(19) Europäisches Patentamt
European Patent Office
Office européen des brevets

(11) Veröffentlichungsnummer: **0 099 985**
**A2**

# (12) EUROPÄISCHE PATENTANMELDUNG

(21) Anmeldenummer: **83105923.3**

(22) Anmeldetag: **16.06.83**

(51) Int. Cl.³: **C 07 D 251/36**

(30) Priorität: **26.07.82 DE 3227817**

(43) Veröffentlichungstag der Anmeldung: **08.02.84 Patentblatt 84/6**

(84) Benannte Vertragsstaaten: **AT BE CH DE FR GB IT LI LU NL SE**

(71) Anmelder: **CHEMIE LINZ AKTIENGESELLSCHAFT, St. Peter-Strasse 25, A-4020 Linz (AT)**

(84) Benannte Vertragsstaaten: **BE CH FR GB IT LI LU NL SE AT**

(71) Anmelder: **Lentia Gesellschaft mit beschränkter Haftung, Arabellastrasse 4 Postfach 81 05 08, D-8000 München 81 (DE)**

(84) Benannte Vertragsstaaten: **DE**

(72) Erfinder: **Schwarz, Franz Thomas, Dipl.-Ing. Dr., Burgerstrasse 82, A-4020 Linz (AT)**
Erfinder: **Lunzer, Friedrich, Dipl.-Ing. Dr., Hostauerstrasse 27, A-4100 Ottensheim (AT)**
Erfinder: **Fierlinger, Adolf, Schumpeterstrasse 2a, A-4045 Linz (AT)**

## (54) Verfahren zur Herstellung von grobkristallinem, rieselfähigem Natriumdichlorisocyanuratdihydrat.

(57) Neues, vorteilhaftes Verfahren zur Herstellung von rieselfähigem, grobkristallinem Natriumdichlorisocyanuratdihydrat durch Umsetzung von Dichlorisocyanursäure und Natriumhydroxid, Natriumcarbonat oder Natriumhydrogencarbonat in einem Reaktionsmedium, welches auf 70 bis 80 Gew.-Teile eines organischen, mit Wasser mischbaren und gegenüber Dichlorisocyanursäure inerten Lösungsmittels 20 bis 30 Gew.-Teile Wasser enthält, und Entfernen des organischen Lösungsmittels zusammen mit noch vorhandenem freiem Wasser durch Trocknen im Vakuum bei Temperaturen von Raumtemperatur bis 50 °C.

EP 0 099 985 A2

Verfahren zur Herstellung von grobkristallinem, rieselfähigem Natriumdichlorisocyanuratdihydrat

Die vorliegende Erfindung betrifft ein neues vorteilhaftes Verfahren zur Herstellung von grobkristallinem und rieselfähigem Natriumdichlorisocyanuratdihydrat.

Natriumdichlorisocyanuratdihydrat kann durch folgende Strukturformel

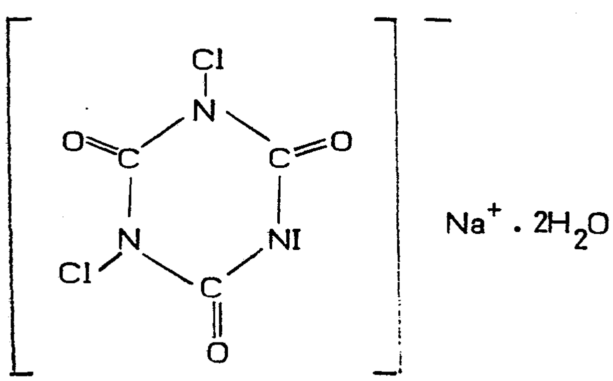

dargestellt werden, wobei aber weder die Strukturformel noch die Bezeichnung Natriumdichlorisocyanuratdihydrat eine Beschränkung auf eine der tautomeren Formen darstellen soll, in der das Salz existieren kann.

Natriumsalze der Dichlorisocyanursäure finden breite Verwendung als Grundstoffe für verfügbares Chlor in festen Bleich-, Sterilisations-, Desinfektions- und Reinigungsmitteln und haben gegenüber herkömmlichen Chlorverbindungen, beispielsweise Chloramin T, 1,3-Dichlor-5,5-dimethylhydantoin, Lithiumhypochlorit oder auch Trichlorisocyanursäure den Vorteil, daß sie unter Umgebungsbedingungen und in Abwesenheit von wesentlichen Feuchtigkeitsmengen bedeutend stabiler sind. Die Natriumsalze der Dichlorisocyanursäure sind daher gut lagerfähig und geben ihr aktives Chlor erst ab, wenn sie in eine wässerige Lösung gebracht werden, die für Bleichwirkung, eine sterilisierende oder desinfizierende Wirkung vorgesehen ist.

Natriumdichlorisocyanurate kommen in drei verschiedenen Formen vor, nämlich in wasserfreier Form, als Monohydrat mit etwa 7,6 Gew.% Hydratationswassergehalt und als Dihydrat mit etwa 14,1 Gew.% Hydratationswassergehalt (vgl. dazu US-PS-3,035.056 und US-PS-3,035.057).

Das Natriumsalz der Dichlorisocyanursäure in der Dihydratform hat gegenüber der wasserfreien Form und dem Monohydrat sowie gegenüber den meisten anderen Ausgangsmaterialien für aktives Chlor den großen Vorteil, daß es weder entzündbar noch brennbar ist. Dadurch kann bei der Herstellung, der Lagerung und dem Versand der oben erwähnten Bleich-, Sterilisations-, Reinigungs- und Desinfektionsmittel auf teure und umständliche Vorsichtsmaßnahmen verzichtet werden.

Gemäß einem in der US-PS-3,035.056 beschriebenen Verfahren wird Natriumdichlorisocyanuratdihydrat durch Behandlung von Trinatriumisocyanurat mit Chlor hergestellt. Bei dieser Umsetzung entsteht ein Gemisch von Natriumdichlorisocyanurat und Natriumchlorid nebeneinander in wässeriger Lösung. Die Anwesenheit von Natriumchlorid neben Natriumdichlorisocyanurat ist jedoch unerwünscht, da die Stabilität der Dihydratsalze durch anhaftendes Natriumchlorid beeinträchtigt wird. Das Reaktionsgemisch wird daher zur Abtrennung von Natriumchlorid mit großen Mengen Wasser verdünnt, was aber zur Folge hat, daß auch beträchtliche Mengen an Natriumdichlorisocyanurat in der Mutterlauge zurückbleiben. Wegen des Gehalts an NaCl kann diese Mutterlauge aber nicht ohne weiteres wieder in die Reaktion zurückgeführt und weiterverwendet werden.

In der DE-PS-1,207.393 ist ein Verfahren zur Herstellung von Natriumdichlorisocyanurat und dessen Mono- und Dihydrat beschrieben, bei dem 2 Äquivalente Trichlorisocyanursäure und 1 Äquivalent Trinatriumcyanurat in wässerigem Medium bei konstantem pH-Wert von 5 - 7 umgesetzt werden. Bei diesem Verfahren wird zwar keine nennenswerte Menge an Natriumchlorid als Nebenprodukt gebildet, es ist jedoch notwendig, Trichlorisocyanursäure als Ausgangsmaterial zusätzlich herzustellen.

Schließlich ist es aus der DE-OS-2,324.356 bekannt, Natriumdichlorisocyanuratdihydrat durch Neutralisation von Dichlorisocyanursäure mit wässeriger Natronlauge bei einem pH-Wert von 6 - 7 und einer Temperatur von 5 bis 65$^{0}$C herzustellen. Nach Beendigung der Umsetzung werden wenigstens 95 % des anhaftenden Wassers durch Trocknen bei 15 bis 50$^{0}$C entfernt, wobei eine Produktenmischung an Dihydrat, Monohydrat und Dichlorisocyanurat mit einem Wassergehalt von etwa 11 Gew.% anfällt.

Alle genannten Verfahren arbeiten in wässerigem Medium, wodurch bei der Abtrennung, Reinigung und Trocknung der Salze erhebliche Schwierigkeiten auftreten.

Das Natriumdichlorisocyanuratdihydrat fällt aus Wasser in feinstkristalliner Form mit einer sehr niedrigen Querschnittfläche an und kann daher nur schwer aus der Mutterlauge abgetrennt und ausgewaschen werden. Nach der Ausscheidung aus der wässerigen Aufschlämmung liegt das Dihydratsalz als feuchte Substanz mit einem Haftwassergehalt von etwa 10 bis 40 Gew.% vor und muß unter schonenden Bedingungen getrocknet werden. Es erweist sich dabei als schwierig, Natriumdichlorisocyanuratdihydrat so zu trocknen, daß es nur vernachlässigbare Mengen an freiem Wasser enthält, ohne daß zugleich das locker gebundene Hydratwasser entfernt wird. Bei höheren Trocknungstemperaturen wird außerdem der Gehalt an aktivem Chlor durch Zersetzung vermindert und es treten empfindliche Verluste durch Sublimation auf. Nach dem Trocknen erhält man ein zum größten Teil staubförmiges Produkt, welches in dieser Form bei der Weiterverarbeitung und Verwendung als Bleichmittel Nachteile bietet.

Weiters fällt bei den bekannten Herstellungsvarianten eine gesättigte wässerige Mutterlauge an, die in Abhängigkeit von der Temperatur, bei der die Herstellung erfolgt, im Bereich zwischen $0^oC$ und etwa $40^oC$ von etwa 10 bis 25 Gew.% gelöstes Natriumdichlorisocyanurat enthält. Es ist daher bei einem wirtschaftlich vertretbaren Verfahren notwendig, durch Eindampfen der Mutterlauge die Ausbeute an Natriumdichlorisocyanuratdihydrat zu verbessern. Während des Eindampfens entstehen aber in der Mutterlauge Hydrolyse- und Zerfallprodukte, welche die Beständigkeit des hergestellten Natriumdichlorisocyanuratdihydrats beeinträchtigen.

Zur Vermeidung dieser Schwierigkeiten ist in der DE-AS-2,433.113 ein Trocknungsverfahren beschrieben, bei dem das Natrimdichlorisocyanuratdihydrat nach dem Abtrennen aus dem wässerigen Reaktionsmedium wieder mit einem mit Wasser mischbaren organischen Lösungsmittel, beispielsweise Aceton, in Kontakt gebracht und bei einer Temperatur von weniger als $40^oC$ getrocknet wird, um das organische Lösungsmittel zusammen mit dem Restgehalt an freiem Wasser zu entfernen. Bei diesem Verfahren erhält man zwar ein rieselfähiges Produkt, nimmt aber den Nachteil in Kauf, daß das Salz zuerst aus dem wässerigen Reaktionsmedium abgetrennt und anschließend wieder mit einem organischen Lösungsmittel vermischt werden muß.

Den in der DE-OS-1,670.985, DE-PS-1,162.378 und DE-AS-1,178.072 angegebenen Verfahren zur Herstellung von chlorierter Cyanursäure oder von deren Salzen ist gemeinsam, daß entweder dem wässerigen Reaktionsmedium oder der abgetrennten Feuchtsubstanz ein oberflächenaktiver Stoff zugesetzt wird. Durch diesen Zusatz erhält man nach dem Trocknen zwar Kristalle mit größerer Querschnittfläche, vermeidet aber nicht die mit dem Trocknen des Haftwassers verbundenen Schwierigkeiten und den hohen Gehalt an Natriumdichlorisocyanurat in der wässerigen Mutterlauge.

Der vorliegenden Erfindung liegt daher die Aufgabe zugrunde, ein wirtschaftliches Verfahren bereitzustellen, das es gestattet, Natriumdichlorisocyanuratdihydrat in grobkristalliner Form unter Vermeidung der den bekannten Verfahren anhaftenden Nachteile herzustellen und auf einfache Weise ohne Entfernung des Hydratwasseranteils unter Erhalt eines rieselfähigen Schüttguts zu trocknen.

Es konnte nun überraschenderweise gefunden werden, daß diese Aufgabe erfindungsgemäß dadurch gelöst werden kann, daß man Dichlorisocyanursäure mit einer basischen Natriumverbindung in einem Reaktionsmedium neutralisiert, welches neben einem organischen, mit Wasser mischbaren Lösungsmittel eine bestimmte, zur Bildung des Dihydratsalzes ausreichende Wassermenge enthält, wobei das Natriumdichlorisocyanuratdihydrat in grobkristalliner, leicht zu trocknender Form anfällt, was einen wesentlichen verfahrenstechnischen und wirtschaftlichen Fortschritt mit sich bringt.

Gegenstand der vorliegenden Erfindung ist demnach ein Verfahren zur Herstellung von grobkristallinem, rieselfähigem Natriumdichlorisocyanuratdihydrat durch Umsetzung von Dichlorisocyanursäure mit einer basischen Natriumverbindung in Gegenwart von Wasser, Abtrennen der festen Reaktionsprodukte und anschließendem Trocknen, das dadurch gekennzeichnet ist, daß äquivalente Mengen an Dichlorisocyanursäure und Natriumhydroxid, Natriumcarbonat oder Natriumhydrogencarbonat bei Temperaturen von $0^{o}C$ bis Raumtemperatur in einem Reaktionsmedium umgesetzt werden, welches auf 70 bis 80 Gew.Teile eines organischen, mit Wasser mischbaren und gegenüber Dichlorisocyanursäure inerten Lösungsmittels 20 bis 30 Gew.Teile Wasser enthält und dessen Menge so bemessen ist, daß mindestens 2 Moläquivalente Wasser bezogen auf die Menge der eingesetzten Dichlorisocyanursäure zugegen sind, worauf das in grobkristalliner Form anfallende Natriumdichlorisocyanuratdihydrat abgetrennt und das organische Lösungsmittel zusammen mit noch anhaftendem freiem Wasser durch Trocknen im Vakuum bei Temperaturen von Raumtemperatur bis $50^{o}C$ entfernt wird.

Das erfindungsgemäße Verfahren kann so ausgeführt werden, daß die Dichlorisocyanursäure im organischen Reaktionsmedium mit einem Wassergehalt von 20 bis 30 Gew.% gelöst oder aufgeschlämmt wird und unter kontinuierlichem Rühren mit der äquivalenten Menge der basischen Natriumverbindung versetzt wird. Es ist aber auch möglich, die Dichlorisocyanursäure im organischen Lösungsmittel zu lösen und unter Rühren mit einer wässerigen Lösung der basischen Natriumverbindung umzusetzen, deren Konzentration so berechnet ist, daß der Wassergehalt der Reaktionsmischung an Neutralisationspunkt 20 bis 30 Gew.Teile bezogen auf die Menge des organischen Lösungsmittels beträgt.

Natriumhydroxyd wird zur Neutralisation der Dichlorisocyanursäure auf jeden Fall bevorzugt in Form von wässeriger Natronlauge zugesetzt. Bei der Berechnung der Konzentration der wässerigen NaOH ist aber der Wassergehalt des organischen Lösungsmittels, in dem die Dichlorisocyanursäure vorgelegt wird, zu beachten, damit am Neutralisationspunkt ein Wassergehalt von 20 bis 30 Gew.Teilen eingehalten wird.

Besonders günstige Reaktionsbedingungen innerhalb der oben angegebenen Grenzen liegen dann vor, wenn man die erfindungsgemäße Umsetzung in Gegenwart von 20 bis 25 Gew.Teilen Wasser bezogen auf die Menge des organischen Lösungsmittels ausführt. Während der Neutralisation ist die Einhaltung einer Reaktionstemperatur von 0 bis $10^{0}C$, insbesondere von 0 bis $5^{0}C$, bevorzugt.

Nach der Vervollständigung der Reaktion, die in Abhängigkeit von der eingesetzten Menge und der Natur der basischen Natriumverbindung von einer bis zu mehreren Stunden dauern kann, wird die Natriumdichlorisocyanuratdihydrat enthaltende Aufschlämmung aus dem Reaktionsgefäß entfernt. Die Festsubstanzen werden von der flüssigen Phase des Umsetzungsgemisches nach irgendeinem bekannten Verfahren zum Abtrennen von Festsubstanzen von Flüssigkeiten, beispielsweise durch Filtrieren, Zentrifugieren, Dekantieren odgl. abgetrennt, wobei das Dihydratsalz der Dichlorisocyanursäure in einer leicht filtrierbaren grobkristallinen Form ausfällt und je nach Verfahrensvariante und Menge des Wassers im organischen Lösungsmittel nur 5 bis 8 Gew.% Restfeuchtigkeit bezogen auf Natriumdichlorisocyanurat enthält. Das vom organischen Lösungsmittel noch feuchte, feste Produkt wird dann zwecks Gewinnung von stabilem, reinem Natriumdichlorisocyanuratdihydrat im Wasserstrahlvakuum bis zur Gewichtskonstanz getrocknet. Es werden dabei Trockentemperaturen von unter $50^{0}C$, vorzugsweise Temperaturen von 25 bis $30^{0}C$ verwendet, wobei die Entfernung des organischen Lösungsmittels zusammen mit den geringen Mengen freien Wassers äußerst schnell und einfach durchzuführen ist. Unter den angegebenen Bedingungen erhält man trockene, freifließende Kristalle von Natriumdichlorisocyanuratdihydrat, ohne daß ein Verlust von Hydratwasser eintritt.

Von den organischen Lösungsmitteln, die mit Wasser mischbar sind und mit Dichlorisocyanursäure nicht reagieren, eignen sich für die Durchführung des erfindungsgemäßen Verfahrens beispielsweise Methanol oder Acetonitril, wobei die Verwendung von Methanol besonders bevorzugt ist. Die Menge an Reaktionsmedium, die verwendet wird, ist so zu bemessen, daß das Reaktionsgemisch über den ganzen Umsetzungszeitraum hin bis zur Entfernung aus dem Reaktionsgefäß gut rührbar und durchmischbar bleibt und daß mindestens 2 Moläquivalente Wasser bezogen auf die Menge der eingesetzten Dichlorisocyanursäure vorhanden sind.

Für die Neutralisation der Dichlorisocyanursäure kommen als basische Natriumverbindungen vor allem Natronlauge, Natriumcarbonat und insbesondere Natriumhydrogencarbonat in Betracht. Der besondere Vorteil bei der Verwendung von Natriumhydrogencarbonat liegt darin, daß durch die Verdampfungskälte des entweichenden Kohlendioxids der Reaktion die Neutralisationswärme laufend entzogen wird und daher keine äußerliche Kühlung während der Umsetzung erforderlich ist.

Eine besonders günstige und wirtschaftliche Ausführungsform des Verfahrens liegt dann vor, wenn die Mutterlauge im Kreislauf geführt wird. In diesem Fall wird nach Beendigung der Neutralisierungsreaktion die Aufschlämmung aus dem Reaktionsgefäß entfernt und nach irgendeinem bekannten Trennungsverfahren in einen festen Produktstrom und flüssigen Abstrom getrennt.

Dieser Abstrom wird wieder in das Reaktionsgefäß zurückgeführt, jeweils mit frischer Dichlorisocyanursäure sowie der äquivalenten Menge an basischer Natriumverbindung nach einer der oben angegebenen Methoden versetzt und auf einen Wassergehalt von 20 bis 30 Gew.% bezogen auf die Menge des organischen Lösungsmittels eingestellt. Dabei ist es von Zeit zu Zeit notwendig, auch den mit dem festen Produkt mitgerissenen organischen Lösungsmittelanteil zu ergänzen. Das mit dem Produktstrom abgetrennte Dihydratsalz der Dichlorisocyanursäure wird zu einem Vakuumtrockner geführt und unter den oben angegebenen Bedingungen getrocknet.

Da in der organischen Mutterlauge bei einer Umsetzungstemperatur von 3°C nur etwa 2 - 4 Gew.% gelöstes Natriumdichlorisocyanurat zurückbleiben, kann die Mutterlauge unmittelbar nach dem Abtrennen der festen Bestandteile ohne Eindampfen wieder der Reaktion zugeführt werden.

Im erfindungsgemäßen Reaktionsmedium werden daher auch nach mehrfacher Rückführung keine Hydrolyseprodukte oder Verunreinigungen beobachtet, welche die Beständigkeit des hergestellten Natriumdichlorisocyanuratdihydrats beeinträchtigen würden.

Die Dichlorisocyanursäure kann als Ausgangsmaterial nach jedem bekannten Verfahren hergestellt und für das erfindungsgemäße Verfahren verwendet werden.

Besonders vorteilhaft ist es aber, Dichlorisocyanursäure durch Umsetzung von Trichlorisocyanursäure mit einem Wassergehalt von etwa 10 Gew.% mit trockener Cyanursäure direkt in dem für die Neutralisation vorgesehenem Reaktionsmedium in homogener Lösung herzustellen. Auf diese Weise wird der Wasserhaushalt in der Mutterlauge durch Zugabe von jeweils frischer wasserhältiger Trichlorisocyanursäure nach Beendigung eines jeden Reaktionszyklus gerade aufrechterhalten.

Das Produkt des erfindungsgemäßen Verfahrens, d.h. Natriumdichlorisocyanuratdihydrat fällt in Form grobkörniger und gut rieselfähiger Kristalle an und hat ausgezeichnete Lagerbeständigkeits- und freifließende Eigenschaften. Das erfindungsgemäße Verfahren hat gegenüber den herkömmlichen Verfahren den Vorteil, daß das Reaktionsmedium ohne Aufarbeitung weiterverwendet werden kann, ohne daß dadurch die Qualität der hergestellten Natriumdichlorisocyanuratdihydrats vermindert wird und das Produkt ohne Abspaltung von Hydratationswassern einfach und schnell getrocknet werden kann.

Die folgenden Beispiele sollen das erfindungsgemäße Verfahren erläutern:

Beispiel 1:

99 g Dichlorisocyanursäure werden in einer Mischung aus 500 g Methanol und 100 g Wasser aufgeschlämmt und auf 3°C gekühlt. Unter Rühren werden innerhalb von 45 Minuten 40 g einer 50 %igen wässerigen NaOH-Lösung zugegeben. Man rührt nach beendigter Alkalizugabe noch 15 Minuten bei 3°C nach, wobei grobkristallines Natriumdichlorisocyanuratdihydrat ausfällt. Das kristalline Produkt (110 g) wird durch Zentrifugieren abgetrennt und anschließend bei 35°C durch zweistündiges Trocknen im Wasserstrahlvakuum vom Lösungsmittel befreit. Man erhält so 102 g grobkristallines, gut rieselfähiges Natriumdichlorisocyanuratdihydrat. Im Filtrat werden weitere 79 g Dichlorisocyanursäure suspendiert und mit 32 g 50 %iger wässeriger NaOH-Lösung versetzt. Nach Beendigung der Neutralisation, Abtrennen und Trocknen, erhält man weitere 102 g Natriumdichlorisocyanuratdihydrat mit einem Wassergehalt von 14,0 Gew.% und einem Gehalt an verfügbarem Chlor von 55,4 %, was etwa einer 100 %igen Ausbeute entspricht.

Beispiel 2:

In 500 g einer bei 25°C gesättigten Lösung von Natriumdichlorisocyanurat in einem Methanol/Wassergemisch, die von einer früheren Umsetzung stammt und einen Wassergehalt von 30 Gew.% bezogen auf die Methanolmenge aufweist, werden 50 g Dichlorisocyanursäure suspendiert und unter Rühren bei 5°C mit 13,4 g fester Soda versetzt. Nach 60 Minuten wird der kristalline Feststoff (70g) abfiltriert und 3 Stunden lang bei 30°C im Wasserstrahlvakuum getrocknet. Auf diese Weise erhält man 64 g Natriumdichlorisocyanuratdihydrat als grobkristallines, rieselfähiges Schüttgut.

Beispiel 3:

In 500 g einer mit Natriumdichlorisocyanurat gesättigten Lösung in Acetonitril /$H_2O$ (80 : 20) werden bei 10°C 50 g Dichlorisocyanursäure vorgelegt und mit 21,2 g festem Natriumhydrogencarbonat versetzt. Ohne weitere Kühlung von außen wird das Reaktionsgemisch zur Vervollständigung der Kristallisation 2 Stunden lang gerührt, der ausgefallene Niederschlag abgetrennt und 3,5

Stunden bei 35°C im Vakuum getrocknet. Es werden so 64 g Natriumdichlorisocyanuratdihydrat in guter Kristallqualität gewonnen.

Beispiel 4:

Zu einer Dichlorisocyanursäure enthaltenden Reaktionsmischung, die dadurch entsteht, daß man in 600 g einer mit Natriumdichlorisocyanurat gesättigten Methanol/Wassermischung (5 : 1) bei 5°C 69 g feuchte Trichlorisocyanursäure mit einem Wassergehalt von 10,4 Gew.% und 17,2 g trockene Cyanursäure unter weitgehender Auflösung aufgeschlämmt und gut durchmischt, werden unter Rühren portionsweise 33,6 g Natriumhydrogencarbonat gegeben. Nach 2-stündiger Reaktionszeit wird das feste Produkt abgetrennt, zu einem Vakuumtrockner gebracht und 4 Stunden bei 25°C getrocknet. Auf diese Weise werden 101 g Natriumdichlorisocyanuratdihydrat in grobkristalliner Qualität mit freifließenden Eigenschaften gewonnen.

Der flüssige Abstrom wird durch Zusatz von 7 g einer Methanol/Wassermischung (5 : 1) ergänzt und in das Reaktionsgefäß zurückgeführt. Nach Zugabe von 69 g Trichlorisocyanursäure mit einem Wassergehalt von 10 Gew.% und 17,2 g trockener Cyanursäure wird mit Natriumhydrogencarbonat unter den oben angegebenen Bedingungen bis zur Neutralisation versetzt. Bei 10maliger Wiederholung werden in jedem Reaktionszyklus jeweils 100 bis 103 g Natriumdichlorisocyanuratdihydrat von gleichbleibend guter Kristallinität und Reinheit gewonnen.

Patentansprüche:

1. Verfahren zur Herstellung von grobkristallinem, rieselfähigem Natriumdichlorisocyanuratdihydrat durch Umsetzung von Dichlorisocyanursäure mit einer basischen Natriumverbindung in Gegenwart von Wasser, Abtrennen der festen Reaktionsprodukte und anschließendem Trocknen, dadurch gekennzeichnet, daß äquivalente Mengen an Dichlorisocyanursäure und Natriumhydroxid, Natriumcarbonat oder Natriumhydrogencarbonat bei Temperaturen von $0^{0}C$ bis Raumtemperatur in einem Reaktionsmedium umgesetzt werden, welches auf 70 bis 80 Gew.Teile eines organischen, mit Wasser mischbaren und gegenüber Dichlorisocyanursäure inerten Lösungsmittels 20 bis 30 Gew.Teilen Wasser enthält und dessen Menge so bemessen ist, daß mindestens 2 Moläquivalente Wasser bezogen auf die Menge der eingesetzten Dichlorisocyanursäure zugegen sind, worauf das in grobkristalliner Form anfallende Natriumdichlorisocyanuratdihydrat abgetrennt und das organische Lösungsmittel zusammen mit noch anhaftendem freiem Wasser durch Trocknen im Vakuum bei Temperaturen von Raumtemperatur bis $50^{0}C$entfernt wird.

2. Verfahren nach Anspruch 1, dadurch gekennzeichnet, daß die Dichlorisocyanursäure im Reaktionsmedium gelöst oder aufgeschlämmt und unter Rühren mit der äquivalenten Menge an basischer Natriumverbindung versetzt wird.

3. Verfahren nach Anspruch 1, dadurch gekennzeichnet, daß die Dichlorisocyanursäure in dem organischen Lösungsmittel, das Teil des Reaktionsmediums ist, gelöst und mit einer wässerigen Lösung der basischen Natriumverbindung umgesetzt wird, deren Konzentration so berechnet ist, daß der Wassergehalt der Reaktionsmischung an Neutralisationspunkt zumindest 20 und höchstens 30 Gew.Teile auf 100 Gew.Teile Reaktionsmedium beträgt.

4. Verfahren nach den Ansprüchen 1 bis 3, dadurch gekennzeichnet, daß man die Umsetzung in einem Reaktionsmedium durchführt, welches auf 100 Gew.Teile 20 bis 25 Gew.Teile Wasser enthält.

5. Verfahren nach den Ansprüchen 1 bis 4, dadurch gekennzeichnet, daß man die Umsetzung bei einer Temperatur von 0 bis 5°C ausführt.

6. Verfahren nach den Ansprüchen 1 bis 5, dadurch gekennzeichnet, daß man als organisches Lösungsmittel Methanol verwendet.

7. Verfahren nach den Ansprüchen 1 bis 6, dadurch gekennzeichnet, daß man für die Neutralisation der Dichlorisocyanursäure Natriumhydrogencarbonat verwendet.

8. Verfahren nach den Ansprüchen 1 bis 7, dadurch gekennzeichnet, daß man nach Beendigung der Umsetzung die Aufschlämmung aus dem Reaktionsgefäß entfernt, in einen festen Produktstrom und flüssigen Abstrom trennt, worauf man letzteren im Kreislauf in das Reaktionsgefäß zurückführt, mit äquivalenten Mengen der Reaktionsteilnehmer versetzt und auf einen Wassergehalt von 20 bis 30 Gew.Teilen pro 100 Gew.Teile Reaktionsmedium einstellt.

O.Z.704
25.5.1983